Europäisches Patentamt

European Patent Office

Office européen des brevets

(19)

(11) **EP 1 344 530 A1**

(12) **EUROPEAN PATENT APPLICATION**
published in accordance with Art. 158(3) EPC

(43) Date of publication:
**17.09.2003 Bulletin 2003/38**

(21) Application number: **02779932.9**

(22) Date of filing: **29.10.2002**

(51) Int Cl.[7]: **A61K 38/01**

(86) International application number:
**PCT/JP02/11228**

(87) International publication number:
**WO 03/037364 (08.05.2003 Gazette 2003/19)**

(84) Designated Contracting States:
**AT BE BG CH CY CZ DE DK EE ES FI FR GB GR
IE IT LI LU MC NL PT SE SK TR**

(30) Priority: **31.10.2001 JP 2001334120**

(71) Applicant: **Morinaga Milk Industry Co., Ltd.
Minato-ku, Tokyo 108-8384 (JP)**

(72) Inventors:
 • **HAYASAWA, H.,
 C/O MORINAGA MILK INDUSTRY CO. LTD
 Zama-shi, Kanagawa 228-8583 (JP)**
 • **TAMURA, Y.,
 C/O MORINAGA MILK INDUSTRY CO. LTD
 Zama-shi, Kanagawa 228-8583 (JP)**

 • **MIYAKAWA, H.,
 C/O MORINAGA MILK INDUSTRY CO. LTD
 Zama-shi, Kanagawa 228-8583 (JP)**
 • **YAMADA, A.,
 C/O MORINAGA MILK INDUSTRY CO. LTD
 Zama-shi, Kanagawa 228-8583 (JP)**
 • **KUHARA, T.,
 C/O MORINAGA MILK INDUSTRY CO. LTD
 Zama--shi, Kanagawa 228-8583 (JP)**
 • **OCHI, H.,
 C/O MORINAGA MILK INDUSTRY CO. LTD
 Zama-shi, Kanagawa 228-8583 (JP)**

(74) Representative: **Bannerman, David Gardner et al
Withers & Rogers,
Goldings House,
2 Hays Lane
London SE1 2HW (GB)**

(54) **INTERLEUKIN-18 INDUCING AGENT**

(57)     Whey protein is hydrolyzed with hydrolase so that a rate of hydrolysis is preferably 20 to 30 %. A fraction, which has molecular weights above 10,000 daltons, is removed from a resultant hydrolysate by means of an ultrafiltration method to obtain a whey protein hydrolysate which is used as an active ingredient of an interleukin-18 inducer.

EP 1 344 530 A1

**Description**

Technical Field

The present invention relates to an interleukin-18 inducer containing a whey protein hydrolysate as an active ingredient.

Background Art

In general, it is acknowledged that specified growth and differentiation factors are required for the growth and the differentiation of hematopoietic cells. A large number of differentiation and growth factors participate in the process to finally arrive at various types of matured haemocytes including, for example, erythrocyte, granulocyte, macrophage, acidophile, platelet, and lymphocyte.

On the other hand, the living body responds to infection of bacteria and viruses, tumor, and cytotoxicity and the like by means of the immune reaction. The immune response is regulated by the direct or indirect interaction between immune cells. In recent years, the immunomodulatory substance, which has an effect to act on the immune function and improve the defensive force against foreign matters, attracts the attention in relation to the cancer therapy.

It has been clarified one after another that the substances, which are generally referred to as "cytokine" including, for example, interleukins, colony stimulating factors, TNF (tumor necrosis factors), and interferons, participate in the differentiation, the growth, and the immune function. Researches have been also made on the method to utilize the cytokine as a medicine by producing the cytokine by means of the genetic engineering technique.

Interleukin-18 (hereinafter sometimes abbreviated as "IL-18") is one of cytokines as information transmitters in the immune system. This cytokine was expressed as an interferon-γ inducing factor at the beginning of the discovery according to, for example, reports described in Japanese Laid-Open Patent Publication Nos. 8-27189, 8-191098, and 9-289896 and *Nature, Vol. 378, No. 6552, pp. 88-91, 1995*. However, this cytokine is called "IL-18" thereafter (*Journal of Immunology, Vol. 156, pp. 4274-4279, 1996*).

Interleukin-18 is structurally a protein belonging to the interleukin-1 family, and it is functionally common to interleukin-12. Interleukin-18 strongly induces the production of interferon-γ from T cells and natural killer cells. Additionally, interleukin-18 has a property to induce granulocyte-macrophage colony-stimulating factor, tumor necrosis factor, interleukin-4, interleukin-5, interleukin-8, interleukin-10, and interleukin-13. Further, interleukin-18 also has a property to enhance the cytotoxicity of natural killer cells and a property to enhance the prolifiration of natural killer cells (*Macrophage Journal, Vol. 6, No. 3, pp. 5-6, 1999* and "Cytokine and Growth Factor, Revised Edition" written by Kohei Miyazono and Kazuo Sugamura, pp. 46-47, Yodosha, 1998). On the other hand, interleukin-18 is considered to have a function to suppress the formation of osteoclast, and it is considered to closely relate to the control of osteoplasty ("New Function of Cytokine and Life Phenomenon", Special Issue of "Experimental Medicine" edited by Atsushi Miyajima, Vol. 18, No. 15, pp. 178-179, Yodosha, 2000). Because of the properties as described above, interleukin-18 is expected to be used in a variety of ways of use as pharmaceutical preparations including, for example, antiallergic agents, antiinflammatory agents, antiviral agents, antimicrobial agents, antitumor agents, and anti-immunological disease agents, about which researches are diligently advanced.

Recent researches include those concerning the strong induction of the production of interferon-γ by interleukin-18 itself. Further, investigations and researches are vigorously performed for the factor to *in vivo* or *in vitro* induce or amplify the cytokine which is believed to exhibit the important function in the therapy and the prevention of immunological diseases. A variety of cytokine inducing factors have been reported. The structures and the functions of the inducing factors have been elucidated, the development has been advanced to prepare pharmaceutical preparations for various diseases, and the products thereof are actually manufactured and utilized in the medical scenes at present.

Disclosure of the Invention

On the contrary, almost all of the inducing factors, which are utilized for the pharmaceutical preparations as described above, involve such a problem that the function can be exhibited only when the pharmaceutical preparation is administered to the living body parenterally, the application of medical treatment is restricted due to the administration method, and any adverse drug action or side effect may be caused.

An object of the present invention is to provide an interleukin-18 inducer which has an effect to be expressed by oral administration, which scarcely causes the adverse drug action, and which has, for example, an antiallergic effect.

As a result of diligent and repeated researches on the influence of milk protein on the production of cytokine during the search for the cytokine inducing factor performed by the inventors of the present invention, it has been found out that a hydrolysate of whey protein has an effect to facilitate the production of interleukin-18. Thus, the present invention has been completed.

The present invention, which solves the problem as described above, is as follows.

(1) An interleukin-18 inducer comprising a whey protein hydrolysate which is obtainable by hydrolyzing whey protein with hydrolase and which has a function to induce interleukin-18, as an active ingredient.
(2) The interleukin-18 inducer as defined in (1), wherein the hydrolase is one or more of those selected from the group consisting of protease originating from *Bacillus subtilis*, protease originating from lactic acid bacteria, and protease originating from animals or plants.
(3) The interleukin-18 inducer as defined in (1) or (2), wherein a rate of hydrolysis of the whey protein hydrolysate is 20 to 30 %.
(4) The interleukin-18 inducer as defined in any one of (1) to (3), wherein a fraction, which has molecular weights above 10,000 daltons, is removed from the whey protein hydrolysate.
(5) The interleukin-18 inducer as defined in (4), wherein the fraction, which has the molecular weights above 10,000 daltons, is removed from the whey protein hydrolysate by means of an ultrafiltration method.
(6) The interleukin-18 inducer as defined in any one of (1) to (5), which contains the whey protein hydrolysate by not less than 0.1 % by weight with respect to a total amount.
(7) A method for producing a whey protein hydrolysate having a function to induce interleukin-18, comprising hydrolyzing whey protein with hydrolase, and removing a fraction having molecular weights above 10,000 daltons from a resultant hydrolysate by means of an ultrafiltration method.

In this specification, the percentage is indicated on the basis of the weight unless otherwise noted, except for the rate of hydrolysis.

The present invention will be specifically explained below.

The interleukin-18 inducer of the present invention comprises a whey protein hydrolysate which is obtainable by hydrolyzing whey protein with hydrolase and which has a function to induce interleukin-18, as an active ingredient. Any material may be used for the whey protein which is used as the starting material for producing the interleukin-18 inducer of the present invention, provided that the material contains the whey protein as a main component. However, it is desirable to use a variety of commercially available whey proteins including, for example, whey protein condensate (WPC) and whey protein isolate (WPI). Alternatively, the whey protein can be purified in accordance with the ordinary method from milk, skim milk, whole milk powder, and skim milk powder as well.

Next, an explanation will be made about the method for hydrolyzing the whey protein with hydrolase. The raw material whey protein is dispersed and dissolved in water or warm water. The concentration of the dissolved solution is not specifically limited. However, in ordinary cases, it is desirable that the concentration is within a range of about 5 to 15 % as calculated on the basis of protein in view of the efficiency and the operability.

It is desirable that the solution containing the whey protein is heated and sterilized at 70 to 90 °C for about 10 minutes to 15 seconds in view of the prevention from deterioration which would be otherwise caused by any contamination with saprophytic bacteria.

Subsequently, it is preferable that an alkaline agent or an acid agent is added to the solution containing the whey protein to adjust pH to the optimum pH for the hydrolase to be used or to pH in the vicinity thereof. As for the alkaline agent or the acid agent to be used for the method of the present invention, any alkaline agent or any acid agent may be used provided that the agent is permitted for food or pharmaceutical preparations. Specifically, the alkaline agent may be exemplified, for example, by sodium hydroxide, potassium hydroxide, and potassium carbonate, and the acid agent may be exemplified, for example, by hydrochloric acid, citric acid, phosphoric acid, and acetic acid.

Subsequently, a hydrolase solution is added to the whey protein solution. The hydrolase is not specifically limited provided that the hydrolase hydrolyzes protein. It is preferable that the hydrolase is endopeptidase. A variety of enzymes can be used as the endopeptidase including, for example, serine protease such as trypsin and elastase, and cysteine protease such as papain and bromelain. In particular, those preferred are exemplified by protease originating from *Bacillus subtilis*, protease originating from lactic acid bacteria, and protease originating from animals and plants. It is noted that the term "originating from" means the fact that the organism described above originally possesses the protease, and the term does not means the source of collection. For example, the protease, which is produced by introducing a gene coding for the protease to be produced by *Bacillus subtilis* into *Escherichia coli* and expressing the gene, "originates from" *Bacillus subtilis*.

For example, commercially available products can be used as the protease originating from *Bacillus subtilis*. Those preferred can be exemplified, for example, by Protease N (produced by Amano Enzyme Inc.) and Bioprase (produced by Nagase Chemtex Corporation). The protease originating from *Bacillus subtilis* is added in a proportion of 100 to 5,000 PUN units per 1 g of the whey protein (the unit will be described later on). As for the PUN unit, 1 PUN unit is represented by the enzyme activity to exhibit the color reaction with the Folin's reagent of allyl amino acid corresponding to 1 μg tyrosine for 1 minute at 30 °C when the protease originating from *Bacillus subtilis* is allowed to act on casein (produced by Merck, Ltd., Hammerstein).

The protease originating from lactic acid bacteria can be produced as follows, for example, in accordance with a method described in the item of "Enzymes to be used" in column 6, line 4 of Japanese Patent Publication No. 54-36235. A lactic acid bacterium is cultivated in accordance with a known method (for example, a method described in Japanese Patent Publication No. 48-43878), and a cell pellet of the lactic acid bacterium is recovered by centrifuging an obtained culture medium. An operation, in which the cell pellet is suspended in sterilized water and centrifugation is performed to recover the cell pellet of the lactic acid bacterium, is repeated twice to wash the cell pellet. The cell pellet is suspended at a concentration of 20 % in sterilized water to disrupt the cell pellet with a cell pellet disrupter (for example, Dyno Mill produced by Willy Bachnfen Engineering Works, KDL type), followed by being lyophilized to obtain a protease powder originating from the lactic acid bacterium. The lactic acid bacterium may be exemplified, for example, by bacteria belonging to the genus *Lactococcus* such as *Lactococcus lactis*, bacteria belonging to the genus *Lactobacillus* such as *Lactobacillus helveticus* and *Lactobacillus bulgaricus*, and bacteria belonging to the genus *Bifidobacterium* such as *Bifidobacterium breve* and *Bifidobacterium longum*.

The enzyme obtained as described above is added at a proportion of 20 to 200 activity units (the unit will be described later on) per 1 g of the whey protein. The activity unit is measured in accordance with the following method. The powder containing the protease is dispersed or dissolved in 0.1 M phosphate buffer (pH 7.0) at a proportion of 0.2 g/100 ml to prepare an enzyme solution. On the other hand, leucinyl-p-nitroanilide (produced by Kokusan Chemical Co., Ltd, hereinafter referred to as "Leu-pNA") is dissolved in 0.1 M phosphate buffer (pH 7.0) to prepare a substrate solution of 2 mM. 1 ml of the substrate solution is added to 1 ml of the enzyme solution to cause the reaction at 37 °C for 5 minutes. After that, 2 ml of 30 % acetic acid solution is added to stop the reaction. The reaction solution is filtrated through a membrane filter, and the absorbance of a resultant filtrate is measured at a wavelength of 410 nm. The amount of enzyme, which is required to decompose 1 μmol of Leu-pNA per 1 minute, is defined as 1 activity unit, and the activity unit of the protease is determined in accordance with the following expression.

$$\text{Activity unit (per 1 g powder)} = 20 \times (A/B)$$

In the expression, A and B represent the absorbance of the sample and the absorbance of 0.25 mM p-nitroaniline at the wavelength of 410 nm respectively.

For example, trypsin, papain, and bromelain can be used as the protease originating from animals and plants.

In the present invention, one hydrolase may be used, or two or more hydrolases may be used. When two or more enzymes are used, the respective enzyme reactions may be performed either simultaneously or separately.

The solution, to which the enzyme is added, is maintained at an appropriate temperature, for example, 30 to 60 °C, desirably 45 to 55 °C depending on the type of the enzyme to start the hydrolysis of the whey protein. As for the reaction time of hydrolysis, the reaction is continued until arrival at a preferred rate of hydrolysis while monitoring the rate of hydrolysis of the enzyme reaction. In order to obtain the whey protein hydrolysate as the active ingredient of the interleukin-18 inducer of the present invention, it is desirable that the rate of hydrolysis is 20 to 30 %.

The following method is available to calculate the rate of hydrolysis of the protein. That is, the total amount of nitrogen of the sample is measured by means of the Kjeldahl method ("Method of Food Analysis", p.102, edited by Japanese Society for Food Science and Technology, Korin Publishing Co., Ltd, 1984), the amount of formol form nitrogen of the sample is measured by means of the formol titration method ("Experiments of Food Engineering", first volume, p. 547, edited by Mitsuda et al., Yokendo, 1970), and the rate of hydrolysis is calculated from the measured values in accordance with the following expression.

$$\text{Rate of hydrolysis} = (\text{formol form nitrogen amount/total nitrogen amount}) \times 100$$

The enzyme reaction is stopped by the deactivation of the enzyme in the hydrolysis solution. The stop of the enzyme reaction can be carried out by means of a heating deactivation treatment in accordance with an ordinary method. As for the heating temperature and the retaining time in the heating deactivation treatment, a condition, under which the deactivation can be sufficiently effected, can be appropriately established taking the thermal stability of the used enzyme into consideration. However, for example, the deactivation can be performed within a temperature range of 80 to 130 °C for a retaining period of time of 30 minutes to 2 seconds.

It is preferable that the obtained reaction solution is adjusted so that pH is within a range of 5.5 to 7 with acid such as citric acid.

It is preferable that the fraction, which has the molecular weights exceeding 10,000 daltons, is removed from the whey protein hydrolysate obtained as described above. The term "remove" includes the fact that a part of the fraction having the molecular weights above 10,000 daltons is removed in addition to the fact that the fraction having the molecular weights above 10,000 daltons is completely removed. However, it is preferable that the fraction is completely removed. No problem arises as far as the interleukin-18-inducing function exists even when the content of high molecular weight components contained in a fraction having molecular weights of not more than 10,000 daltons is lowered as a result of the removal of the fraction having the molecular weights exceeding 10,000 daltons. The fraction, which has the molecular weights above 10,000 daltons, can be removed, for example, by means of the ultrafiltration method. The ultrafiltration method can be carried out by using a known apparatus (for example, ultrafiltration module (produced by Asahi Kasei Corporation)).

The obtained solution containing the whey protein hydrolysate can be used as it is as well. Alternatively, if necessary, it is also possible to use a concentrated solution obtained by concentrating the solution by means of a known method, and a powder obtained by drying the concentrated solution by means of a known method.

The whey protein hydrolysate obtained as described above has the function to induce interleukin-18. Therefore, the condition, under which the whey protein hydrolysate is produced, can be appropriately established by using the index of the function to induce interleukin-18. In the present invention, the term "induction of interleukin-18" means the induction of the production of interleukin-18 by interleukin-18-producing cells.

It is considered that interleukin-18 is produced by biological tissues and cells in organisms including, for example, macrophages such as kupffer cells, intestinal epithelial cells, and epidermic keratinocyte. In particular, the amount of production of interleukin-18 is conspicuously large in the intestinal epithelial tissue. It is considered that the intestinal epithelial cells contribute to the greater part of the production of interleukin-18 *in vivo*. Therefore, the production of interleukin-18, which is caused by the interleukin-18 inducer of the present invention, can be confirmed more conveniently by producing interleukin-18 by using a cell line in which the environment concerning the expression system in the biological tissue is reproducible. The cell line, which is used to produce interleukin-18, is preferably epithelial cells originating from the colon. It is possible to use, for example, cell lines such as Caco-2, COLO205, SK-CO-1, SW48, T84, HT-29, HCT-15, and LS180.

The production of interleukin-18 can be carried out by adding the interleukin-18 inducer of the present invention to a culture system of the cell line to perform the cocultivation. In this procedure, it is preferable that the whey protein hydrolysate as the active ingredient is contained by at least 1 µg/ml in the culture system. When the cell line is cultured, it is possible to use an appropriate commercially available medium such as RPMI-1640 medium and Dulbecco's Modified Eagle's medium (hereinafter abbreviated as "DMEM medium", if necessary). In particular, the cultivation can be continued in a well-suited manner by using a mixed medium of Dulbecco's Modified Eagle's medium/ Ham's F12 medium (hereinafter abbreviated as "DMEM/F12 medium", if necessary). The medium as described above can be used by adding about 5 to 20 % human serum, bovine serum, or fetal bovine serum. If necessary, it is possible to add, for example, human serum albumin, bovine serum albumin, antibiotics, antibody, and 2-mercaptoethanol.

As for the produced interleukin-18, the interleukin-18, which separated by polyacrylamide gel electrophoresis, can be detected by means of the Western blotting method based on the use of anti-IL-18 antibody.

It is known that the immunoglobulin E (IgE) antibody generally causes allergic symptoms such as bronchial asthma, allergic rhinitis, atopic dermatitis, and food allergy. On the other hand, it is known that interleukin-18 suppresses the production of IgE antibody *in vivo* ("Immune Reviews 2001", Special Issue of "Experimental Medicine" edited by Hajime Toriyama, Vol. 19, No. 5, p. 189, Yodosha, 2001, *Clinical Immunology, Vol. 30, pp. 191-198, 1998, Kagaku-Hyoron*, 1998). Accordingly, the effect to induce interleukin-18, which is brought about *in vivo* by the oral administration of the interleukin-18 inducer of the present invention, can be also confirmed by investigating the effect to suppress the production of IgE antibody.

On the other hand, the effect of interleukin-18 to suppress the production of IgE antibody is considered to be achieved by preferentially inducing Th1 in the balance between Th1 and Th2 as two classes of helper T cells. It has been proved that the production of IgG1 antibody and IgG2a antibody is reflected as the index of the balance between Th1 and Th2 *in vivo* (*Cell Technology, Vol. 19, No. 12, pp. 1828-1835, 2000, Clinical immunology, Vol. 30, pp. 191-198, 1998, Kagaku-Hyoron*, 1998). The effect to induce interleukin-18 can be judged by measuring the ratio between the concentrations of the antibodies in blood.

The whey protein hydrolysate, which is the active ingredient of the interleukin-18 inducer of the present invention, is a natural product originating from milk, and hence it has high stability when it is ingested. The whey protein hydrolysate is contained in food such as cow's milk, and it is daily ingested. The whey protein hydrolysate exhibits no toxicity, and any adverse drug action or side effect is scarcely observed when it is continuously ingested over a long period of time. Therefore, the whey protein hydrolysate can be appropriately used by means of any administration method such as oral administration. The whey protein hydrolysate can be processed, for example, into tablets, capsules, troches, syrups, granules, powders, and ointments in accordance with known methods as well. When the drug or medicine is prepared, it is possible to use components such as excipients or fillers, pH-adjusting agents, coloring agents, and flavors or correctives which are ordinarily used for the drug preparation, in addition to the whey protein hydrolysate. Further, it is also possible to use, in combination, any agent having the function to induce interleukin-18 which is known or which will be found out in future.

The whey protein hydrolysate may be also contained as the active ingredient in food and processed as food having the function of the effect to induce interleukin-18 as an embodiment of the interleukin-18 inducer.

As for the interleukin-18 inducer of the present invention, the whey protein hydrolysate, which is the active ingredient necessary to exhibit the effect to induce interleukin-18, may be blended in the following blending amount. That is, as for the blending amount in the interleukin-18 inducer, the active ingredient is preferably contained by at least 0.1 mass % with respect to the final composition.

The amount of administration of the interleukin-18 inducer of the present invention differs depending on, for example, the age and the symptom. However, in order to exhibit the effect to induce interleukin-18, it is preferable that the interleukin-18 inducer is administered at least at a proportion of 100 mg/kg body weight/day.

Best Mode for Carrying Out the Invention

The present invention will be explained more specifically below with reference to Examples. However, the present invention is not limited to Examples described below.

Example 1: Preparation of Whey Protein Hydrolysate

10 g of a commercially available whey protein condensate (protein content: 83 %, produced by Arla Foods Ingredients) was dissolved in 90 g of purified water, to which 106 mg of sodium hydroxide (produced by San-Ei Gen F. F. I., Inc.) was added to prepare a whey protein solution in which pH was adjusted to 8.5. 14,940 activity units (1,800 activity units per lg of protein) of Protease N (produced by Amano Enzyme Inc.), 1,660 activity units (200 activity units per 1 g of protein) of Bioprase (produced by Nagase Chemtex Corporation), 830 activity units (100 activity units per 1 g of protein) of disrupted cell pellet of *Lactobacillus helveticus*, and 10,375 activity units (1,250 activity units per 1 g of protein) of trypsin (produced by Novozymes) were added to the whey protein solution, and the temperature was kept at 50 °C to cause the hydrolysis. The enzyme reaction was monitored on the basis of the rate of hydrolysis. The solution was heated to 85 °C for 10 minutes at a point of time at which the rate of hydrolysis arrived at 25.7 % to deactivate the enzymes. The solution was cooled to 10 °C to prepare a whey protein hydrolysate. The whey protein hydrolysate was treated with an ultrafiltration membrane module (produced by Asahi Kasei Corporation, Code No. SLP-0053) specified by the fractioning molecular weight of 10,000 to recover a fraction filtrated through the membrane. The fraction was lyophilized to obtain about 8.3 g of powdery whey protein hydrolysate.

Example 2: Production of Interleukin-18 Inducer (1)

20 g of crystalline cellulose (produced by Wako Pure Chemical Industries) was collected in a mortar having a capacity of 1,000 ml (produced by Nakashima Seisakusho Co., Ltd.), to which 20 ml of water was added to effect incorporation. Subsequently, 25 g of lactose (produced by Meggle Gmbh) previously sieved with a 48-mesh sieve (produced by Wakamori) was added, and 55 g of the whey protein hydrolysate prepared in Example 1 was added to effect incorporation. An obtained wet material was placed on a 20-mesh sieve made of stainless steel (produced by Wakamori), and the material was extruded by hand onto a stainless steel plate for drying to form granules which were quickly distributed uniformly, followed by being placed in a drying machine to be dried at 25 °C for 2 days to obtain minute granules. The granules were sieved with a 20-mesh sieve made of polyethylene (produced by Wakamori). Granules, which had passed through the sieve, were spread over a wide paper sheet. 2 g of magnesium stearate (produced by Kanto Kagaku), which had been previously sieved with a 48-mesh sieve, was added thereto, followed by being mixed by hand to obtain a homogeneous mixture. The mixture was tableted with a tablet machine (produced by Kimura Seisakusho, KT-2 type) by using an R-bar having a diameter of 8 mm while establishing the compressive pressure under such a condition that the number of forming tablets was 10, the weight of tablet was 6.2 g, and the Monsanto hardness was 3.5 to 5.0 kg to obtain 16 pieces of tablets having the effect to induce interleukin-18 containing about 50 % of the whey protein hydrolysate.

Example 3: Production of Interleukin-18 Inducer (2)

600 g of lactose (produced by Meggle Gmbh), 400 g of corn starch (produced by Nissin Seifun), 400 g of crystalline cellulose (produced by Wako Pure Chemical Industries), and 600 g of the whey protein hydrolysate prepared in Example 1 were sieved with a 50-mesh sieve (produced by Yamato Scientific Co., Ltd.) to obtain a powdery material which was placed in a bag made of polyethylene having a thickness of 0.5 mm, followed by being mixed by means of inversion or upside down, and the powder was charged to capsules (produced by Japan Elanco, No. 1 gelatin capsule, Op. Yellow No. 6 Body, empty weight: 75 mg) to have contents of 275 mg by using a fully automatic capsule filler machine (produced by Sesser Pedini, press type) to obtain 7,000 pieces of capsules having the effect to induce interleukin-18.

Exemplary Test 1

In this test, the effect to induce interleukin-18 was investigated.

(1) Test samples

The whey protein hydrolysate produced in Example 1 was diluted with a solution of DMEM/F-12 medium (produced by ICN Biomedicals, Inc., Catalogue No. 1-800-854-0530) containing 5 % fetal bovine serum (produced by GIBCO) so that the concentrations were 0.1 μg/ml, 1 μg/ml, 10 μg/ml, and 100 μg/ml to prepare Sample 1, Sample 2, Sample 3, and Sample 4. Bovine serum albumin was diluted with the solution of DMEM/F-12 medium containing 5 % fetal bovine serum so that the concentration was 100 μg/ml to prepare a control sample. Further, a casein hydrolysate, which was obtained by hydrolysis while changing the whey protein condensate of Example 1 to casein, was diluted with the solution of DMEM/F-12 medium containing 5 % fetal bovine serum so that the concentration was 100 μg/ml to prepare a milk protein control sample.
An intestinal epithelial cell line Caco-2 (ATCC HTB-37, obtained from American Type Culture Collection) was used as the cells to be used for inducing interleukin-18.

(2) Test method

The intestinal epithelial cell line Caco-2 was suspended by using the DMEM/F-12 medium containing 5 % fetal bovine serum so that the number of cells was $1 \times 10^4$ to prepare a cell solution. 1 ml of the cell solution was added to 12-well culture plate (produced by Falcon) to perform the cultivation for 6 days until the cells arrived at the confluent state. After that, the wells were washed twice with the DMEM/F-12 medium solution, to which 1 ml of each of Samples 1 to 4, the control sample, the milk protein control sample, and the DMEM/F-12 medium solution containing 5 % fetal bovine serum to further continue the cultivation for 8 hours. After the cultivation, a culture supernatant was removed by the aspiration with an aspirator, and the cells were washed twice with Dulbecco's phosphate buffer solution (hereinafter abbreviated as "PBS") at 4 °C. After that, the cells were lysed with 0.2 ml of PBS solution containing 0.1 % sodium dodecyl sulfate (hereinafter abbreviated as "SDS"), 1 % NP40, 0.5 % sodium deoxycholate, and 0.5 mM phenylmethanesulfonyl fluoride. The cell lysate was centrifuged at 10,000 rpm for 10 minutes at 4 °C to recover the supernatant. In this procedure, the supernatants of lysed cells cultured by adding Samples 1 to 4, the control sample, the milk protein control sample, and the DMEM/F-12 medium solution containing 5 % fetal bovine serum respectively were designated as Sample groups 1 to 4, control group, milk protein control group, and negative group respectively.
The protein was quantitated for the respective groups. Amounts corresponding to 10 μg of protein of the respective groups were used to perform electrophoresis in a nonreducing state by using 16 % SDS polyacrylamide electrophoresis gel (produced by TEFCO). After the electrophoresis, the proteins separated in the gel were transferred to a blotting membrane (produced by Amersham Pharmacia, trade name: Hybond-P). The blotting membrane was subjected to a blocking treatment with a solution of skim milk (produced by DIFCO) diluted with PBS so that the concentration was 5 %. After that, a primary antibody treatment was performed at 4 °C for 12 hours with 0.5 μg/ml of human interleukin-18 antibody (produced by MBL). After that, the blotting membrane was washed, and a secondary antibody treatment was performed for 1 hour with a solution of 5,000-fold diluted peroxidase-labeled anti-mouse IgG (produced by MBL). After the secondary antibody treatment, the blotting membrane was washed, and the blotting membrane was treated with a fluorescent substrate solution of ECL Plus (produced by Amersham Pharmacia). Immediately thereafter, the fluorescent reaction was detected by using an X-ray film. A detected image was analyzed and processed with a densitometer to obtain numerical values. The activity to induce interleukin-18 of each of the groups was calculated as a relative activity on condition that the activity of the negative group was 100 %.

(3) Test results

Results of this test are shown in Table 1. Table 1 shows the activities to induce interleukin-18 of the respective groups. As clarified from Table 1, a tendency was confirmed from the sample groups 1 to 4 such that the activity to induce interleukin-18 was increased as the concentration of the whey protein hydrolysate was raised. Further, it was confirmed that the activity to induce interleukin-18 was exhibited when the whey protein hydrolysate was contained by at least 1 μg/ml in the culture medium, under the condition described above. The activity to facilitate the production of interleukin-18 was absent in the bovine serum albumin as the control group and the casein hydrolysate as the milk protein control group. According to this fact, it was revealed in this test that the activity to induce interleukin-18 was brought about by the whey protein hydrolysate.

Table 1

| Test group | IL-18-inducing activity (%) |
| --- | --- |
| Sample group 1 | 95.7 |
| Sample group 2 | 125.0 |
| Sample group 3 | 147.7 |
| Sample group 4 | 214.7 |
| Control group | 90.3 |
| Milk protein control group | 92.3 |

Exemplary Test 2

This test was performed in order to investigate the effective administration amount of the interleukin-18 inducer of the present invention by using the amount of IgE antibody in serum as an index.

(1) Test samples

A solution, which was obtained by dissolving the whey protein hydrolysate produced in Example 1 in physiological saline (produced by Otsuka Pharmaceutical Co., Ltd.) at a concentration of 30 mg/ml, was used. The physiological saline (produced by Otsuka Pharmaceutical Co., Ltd.) was used for a blank test.

(2) Test method

Fifty BALB/c male mice (purchased from Charles River Japan), which were six weeks old and which were randomly classified into test groups each composed of ten mice, were used as test animals.

2.5 µg of ovalbumin (produced by Sigma) was used as an antigen, which was adsorbed to 1 mg of aluminum hydroxide (produced by Wako Pure Chemical Industries) as an adjuvant to perform the intraperitoneal administration to the respective test animals. The animals were left to stand for 3 days after the administration. The samples were forcibly administered continuously for 3 days by using an oral sonde once a day, while the amounts of administration of the whey protein hydrolysate in the samples were 0 mg/kg body weight/day (blank test), 30 mg/kg body weight/day, 100 mg/kg body weight/day, and 300 mg/kg body weight/day for the respective test groups. The animals were left to stand for 4 days, and the samples were continuously administered again for 3 days. The animals were left to stand for 1 day, and then the blood was collected from the hearts of mice to obtain serum samples.

The IgE concentrations in the respective serum samples were measured by means of the ELISA method. That is, a solution of anti-mouse IgE monoclonal antibody R35-92 (produced by PharMingen) as a primary antibody was added to 96-well microplate (produced by Nunc), followed by being adsorbed overnight at 4 °C. Subsequently, bovine serum albumin (produced by Sigma) was used to perform a blocking treatment, and then the serum sample was added to cause the reaction. Subsequently, a solution of biotin-labeled anti-mouse IgE monoclonal antibody R35-118 (produced by PharMingen) was added as a secondary antibody to cause the reaction, and then peroxidase-labeled avidin D (produced by Vector Laboratories) was added to cause the reaction. A peroxidase substrate solution (produced by Kirkegaard & Perry Laboratories, Inc.) as a color development buffer was added to develop the color. The absorbance at 405 nm of the reaction solution was measured to determine the IgE concentration. An average value of those of the ten animals of each of the test groups was calculated to investigate the IgE concentration in the serum for every test group.

(3) Test results

Results of this test are shown in Table 2. Table 2 shows the relationship between the amount of administration of the interleukin-18 inducer and the IgE concentration. As clarified from Table 2, the whey protein hydrolysate as the active ingredient exhibited the remarkable effect to suppress the production of the IgE antibody by administering the whey protein hydrolysate at least at the proportion of 100 mg/kg body weight/day. It is considered that this result indicates the fact that interleukin-18, which has the effect to suppress the production of the IgE antibody, is effectively induced *in vivo*.

Table 2

| Amount of administration of interleukin-18 inducer (mg/kg body weight/day) | IgE concentration (ng/ml) |
|---|---|
| 0 | 3965.2 |
| 30 | 3543.1 |
| 100 | 2684.3 |
| 300 | 2015.1 |

Exemplary Test 3

This test was performed in order to investigate the effective administration amount of the interleukin-18 inducer of the present invention by using the antibody concentration ratio between IgG1 and IgG2a in serum as an index.

(1) Test samples

A solution, which was obtained by dissolving the whey protein hydrolysate produced in Example 1 in physiological saline (produced by Otsuka Pharmaceutical Co., Ltd.) at a concentration of 30 mg/ml, was used. The physiological saline (produced by Otsuka Pharmaceutical Co., Ltd.) was used for a blank test.

(2) Test method

The IgG1 concentration and the IgG2a concentration in the respective serum samples were measured by means of the ELISA method in accordance with the same method as the test method used in Exemplary Test 2 to investigate the IgG1/IgG2a concentration ratio except that a solution of anti-mouse IgG1 monoclonal antibody A85-3 (produced by PharMingen) and a solution of anti-mouse IgG2a monoclonal antibody R11-89 (produced by PharMingen) were used as primary antibodies, and a solution of biotin-labeled anti-mouse IgG1 monoclonal antibody A85-1 (produced by PharMingen) and a solution of biotin-labeled anti-mouse IgG2a monoclonal antibody R19-15 (produced by PharMingen) were used as secondary antibodies.

(3) Test results

Results of this test are shown in Table 3. Table 3 shows the relationship between the amount of administration of the interleukin-18 inducer and the concentration ratio of IgG1/IgG2a. As clarified from Table 3, the concentration ratio of IgG1/IgG2a was decreased as the amount of administration of the interleukin-18 inducer of the present invention was increased. A significant effect was confirmed at not less than the administration amount of 100 mg/kg body weight/day. This effect indicates the fact that the environment, in which Th1 is preferential by the aid of the induction of the production of interleukin-18, is induced. The optimum administration amount coincided with the result of Exemplary Test 2.

Table 3

| Amount of administration of interleukin-18 inducer (mg/kg body weight/day) | IgG1/IgG2a concentration ratio |
|---|---|
| 0 | 0.664 |
| 30 | 0.593 |
| 100 | 0.435 |
| 300 | 0.419 |

Industrial Applicability

As described in detail above, the present invention relates to the interleukin-18 inducer containing the active ingredient of the whey protein hydrolysate. The following effects are brought about by the present invention.

(1) It is possible to effectively induce interleukin-18.
(2) The adverse drug action is scarcely caused, because the interleukin-18 inducer originates from the milk protein.

The interleukin-18 inducer can be used continuously over a long period of time.

(3) The interleukin-18 inducer can be administered orally. The interleukin-18 inducer is convenient and extremely versatile, for example, as compared with the conventional injection.

(4) The interleukin-18 inducer is obtained from the relatively inexpensive raw material such as cow's milk, and the interleukin-18 inducer is mass-producible.

**Claims**

1. An interleukin-18 inducer comprising a whey protein hydrolysate which is obtainable by hydrolyzing whey protein with hydrolase and which has a function to induce interleukin-18, as an active ingredient.

2. The interleukin-18 inducer according to claim 1, wherein the hydrolase is one or more of those selected from the group consisting of protease originating from *Bacillus subtilis*, protease originating from lactic acid bacteria, and protease originating from animals or plants.

3. The interleukin-18 inducer according to claim 1 or 2, wherein a rate of hydrolysis of the whey protein hydrolysate is 20 to 30 %.

4. The interleukin-18 inducer according to any one of claims 1 to 3, wherein a fraction, which has molecular weights above 10,000 daltons, is removed from the whey protein hydrolysate.

5. The interleukin-18 inducer according to claim 4, wherein the fraction, which has the molecular weights above 10,000 daltons, is removed from the whey protein hydrolysate by means of an ultrafiltration method.

6. The interleukin-18 inducer according to any one of claims 1 to 5, which contains the whey protein hydrolysate by not less than 0.1 % by weight with respect to a total amount.

7. A method for producing a whey protein hydrolysate having a function to induce interleukin-18, comprising hydrolyzing whey protein with hydrolase, and removing a fraction having molecular weights above 10,000 daltons from a resultant hydrolysate by means of an ultrafiltration method.

## INTERNATIONAL SEARCH REPORT

| | International application No. |
|---|---|
| | PCT/JP02/11228 |

**A. CLASSIFICATION OF SUBJECT MATTER**
Int.Cl⁷ A61K38/01

According to International Patent Classification (IPC) or to both national classification and IPC

**B. FIELDS SEARCHED**

Minimum documentation searched (classification system followed by classification symbols)
Int.Cl⁷ A61K38/01

Documentation searched other than minimum documentation to the extent that such documents are included in the fields searched

Electronic data base consulted during the international search (name of data base and, where practicable, search terms used)
CA(STN), MEDLINE(STN), EMBASE(STN), BIOSIS(STN)

**C. DOCUMENTS CONSIDERED TO BE RELEVANT**

| Category* | Citation of document, with indication, where appropriate, of the relevant passages | Relevant to claim No. |
|---|---|---|
| A | JP 11-35468 A (Meiji Milk Products Co., Ltd.), 09 February, 1999 (09.02.99), Full text; particularly, Par. Nos. [0018], [0034] (Family: none) | 1-7 |
| A | JP 5-9124 A (The Calpis Food Industry Co., Ltd.), 19 January, 1993 (19.01.93), Full text (Family: none) | 1-7 |
| A | S.PECQUET et al., Immunoglobulin E suppression and cytokine modulationin mice orally tolerized to β-lactoglobulin, Immunology, 1999, Vol.96, pages 278 to 285 | 1-7 |

☐ Further documents are listed in the continuation of Box C.  ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 07 January, 2003 (07.01.03) | 21 January, 2003 (21.01.03) |

| Name and mailing address of the ISA/ | Authorized officer |
|---|---|
| Japanese Patent Office | |
| Facsimile No. | Telephone No. |

Form PCT/ISA/210 (second sheet) (July 1998)